(19) Europäisches Patentamt
European Patent Office
Office européen des brevets

(11) Publication number: **0 204 440 B1**

(12) **EUROPEAN PATENT SPECIFICATION**

(45) Date of publication of patent specification: **12.12.90**

(51) Int. Cl.[5]: **C 07 F 7/10**

(21) Application number: **86303546.5**

(22) Date of filing: **09.05.86**

(54) **Azetidine derivatives production.**

(30) Priority: **09.05.85 JP 99255/85**

(43) Date of publication of application: **10.12.86 Bulletin 86/50**

(45) Publication of the grant of the patent: **12.12.90 Bulletin 90/50**

(84) Designated Contracting States: **CH DE FR GB IT LI**

(56) References cited:
**EP-A- 66 301**
**EP-A-0 078 026**
**EP-A-0 123 444**
**TETRAHEDRON LETTERS, vol. 23, no. 21, 1982, Pergamon Press, GB; P.J. REIDER et al.: Total synthesis of thienamycin: a new approach from aspartic acid"**
**Aldrich Catalogue for 1988/89 pages 275 and 378**
**The file contains technical information submitted after the application was filed and not included in this specification**

(73) Proprietor: **YAMANOUCHI PHARMACEUTICAL CO. LTD.**
**No. 3-11 Nihonbashi-Honcho, 2-chome Chuo-ku Tokyo (JP)**

(72) Inventor: **Tamura, Toshinari**
**No.1-21-10, Midori-cho**
**Hasuda-shi Saitama (JP)**
Inventor: **Yoshida, Makoto**
**No. 2-14-3, Nakahara-cho**
**Kawagoe-shi Saitama (JP)**
Inventor: **Tamazawa, Kazuharu**
**No. 1013-2, Oaza Kaminoda Shiraoka-cho**
**Minami Saitama-gun Saitama (JP)**
Inventor: **Iwamoto, Hidenori**
**No.3-4-5-203, Sakurada Washimiya-cho**
**Kitakatsushika-gun Saitama (JP)**

(74) Representative: **Geering, Keith Edwin et al**
**REDDIE & GROSE 16 Theobalds Road**
**London WC1X 8PL (GB)**

**EP 0 204 440 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to the preparation of 1-substituted silyl-4-oxo-2-azetidine-carboxylic acid lower alkyl esters of general formula (I)

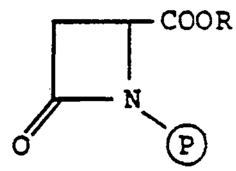

(I)

wherein R represents a lower alkyl group and Ⓟ represents a substituted silyl group.

The compounds (I) are useful as intermediates for producing monobactam compounds having excellent antibacterial activity, for example, 4-alkoxy-carbonyl-2-oxoazetidine-1-sulfonic acid derivatives of formula:

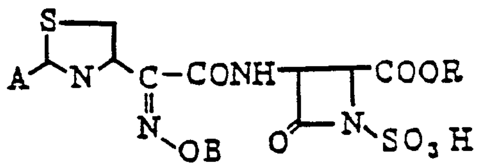

wherein A represents an amino group which may be substituted, B represents a lower alkyl group etc., and R represents a lower alkyl group, described in Published Unexamined Japanese Patent Application No. 131758/82.

The compounds (I) can be converted to useful monobactam compounds via the following steps:

step 1 — introduction of an azido or nitroso group at the 3-position of compound (I);

step 2 — reduction to produce 3-amino-1-substituted silyl-4-oxo-2-azetidine-carboxylic acid lower alkyl ester;

step 3 — acylation of the amino group with carboxylic acid of formula

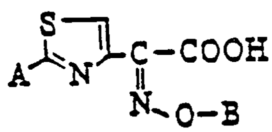

or reactive derivative thereof;

step 4 — desilylation; and

step 5 — sulfonation at the 1-position.

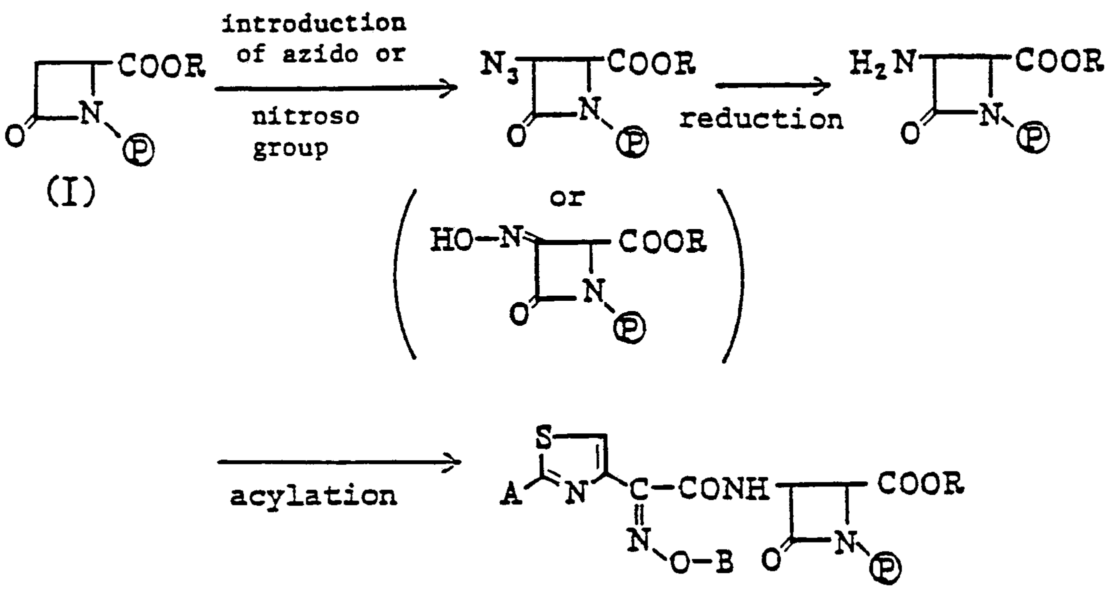

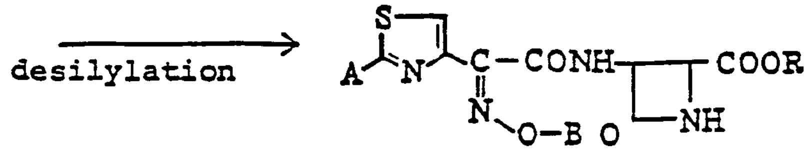


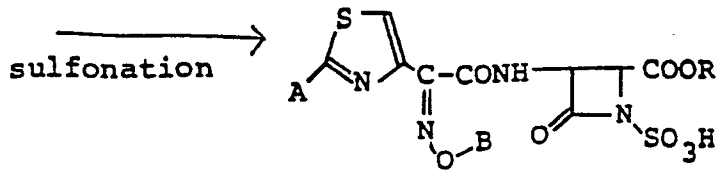


The compounds (I) are novel compounds characterized in chemical structure by the nitrogen at the 1-position being silylated and the carboxylic acid being esterified with a lower alkyl group. Carboxylic acid:

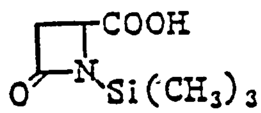

corresponding to the compounds (I), and the benzyl ester:

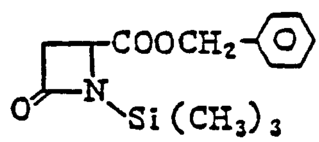

are known (Published Unexamined Japanese Patent Application No. 103358/83). In order to produce the former compound, however, complicated operations for isolation and purification are required. Further, the latter compound has disadvantage in the transformation of an azido or nitroso group introduced in the position 3 to an amino group, because the benzyl ester group is unstable under the conditions of catalytic reduction. The compounds (I) eliminate the foregoing drawbacks and can be stably isolated and are thus advantageous as intermediates for monobactam compounds. In addition, the compounds (I) can be used as intermediates for carbapenems such as thienamycin, etc. and various peptides, e.g., $N^{\alpha}$-[[(S)-4-oxo-2-azetidinyl]carbonyl]-L-histidyl-L-prolinamide useful as a cerebral function activator (Published Unexamined Japanese Patent Application No. 225182/84) and EP 0123444A).

EP—B—0066301 demonstrates in its Example 1 that the above-mentioned prior benzyl ester is unstable, the trimethylsilyl protective group being eliminated in the course of isolation or reaction of the benzyl compound.

The "lower alkyl" groups herein are those having 1 to 5 carbon atoms such as a methyl group, an ethyl group, a propyl group, a butyl group, an isopropyl group, a t-butyl group, a pentyl group, etc. Further, the substituted silyl groups Ⓟ are silyl protective groups tri-substituted with lower alkyl and/or aryl group(s); for example, there may be used a tri-lower alkyl-(or aryl-)silyl group, such as a trimethyl-silyl group, a t-butyldimethylsilyl group, a dimethyl-isobutylsilyl group, a diphenylmethylsilyl group, etc.

The compounds of formula (I) possess an asymmetric carbon and stereoisomers based thereon exist. The compounds (I) include both optically active and racemic forms.

Representative examples of the compounds of formula (I) include the following:

(a) Ethyl (S)-1-trimethylsilyl-4-oxo-2-azetidine-carboxylate;
(b) Methyl (S)-1-trimethylsilyl-4-oxo-2-azetidine-carboxylate;
(c) Ethyl (S)-1-t-butyl-dimethylsilyl-4-oxo-2-azetidine-carboxylate;
(d) Ethyl (S)-1-dimethylisobutylsilyl-4-oxo-2-azetidine-carboxylate;
(e) Ethyl (S)-1-diphenylmethylsilyl-4-oxo-2-azetidine-carboxylate; and
(f) Ethyl (R)-1-trimethylsilyl-4-oxo-2-azetidine-carboxylate.

The compounds (I) are prepared according to the present invention by the following reaction scheme:

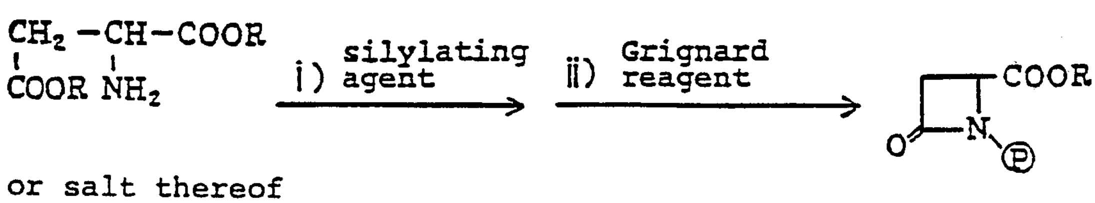


or salt thereof

(II) (I)

This process of the present invention can be performed using aspartic acid di-lower alkyl esters (II) or salts thereof as raw materials, i.e. by silylating the amino group of the raw materials and then converting into β-lactams by Grignard reaction.

When salts of the compounds (II) are used as raw materials, it is preferred that they be silylated after neutralization with bases (preferably, tertiary amines such as triethyl amine, etc.).

i) Silylation

The starting compounds (II) or salts thereof are dissolved in suitable organic solvent such as tetrahydrofuran (THF), toluene, etc. and silylating agent is added to the solution preferably in the presence of amines (triethyl amine, etc.). As the silyating agent, a tri-lower alkyl- or triaryl-silyl halide (chloride, bromide, etc.) is used. The reaction is carried out at room temperature or under cooling, preferably under cooling at less than −10°C.

ii) Grignard Reaction

The Grignard reaction is performed by reacting a Grignard reagent (general formula R'MGX wherein R' represents an alkyl group such as a methyl group, an ethyl group, a t-butyl group, etc. and X represents a halogen atom) under cooling or at room temperature in a solvent (ether, THF, a solvent mixture of THF and toluene, etc.) under anhydrous conditions. It is preferred that the reaction be carried out under an atmosphere of an inert gas (argon, etc).

Isolation of the desired compounds can easily be performed by extracting with solvent, e.g., ethyl acetate. When the desired compounds are used as raw materials for other compounds, they can be used without purification.

In the thus obtained desired compounds, the silyl group at the 1-position thereof can also be converted by desilylating and then racting with other silylating agent:

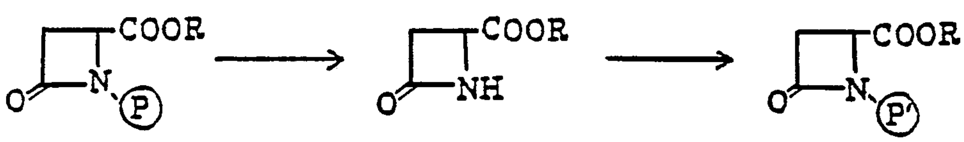

wherein (P') represents a substituted silyl group different from (P).

The production of compounds (I) by the present invention is illustrated in more detail in the Examples below.

Example 1

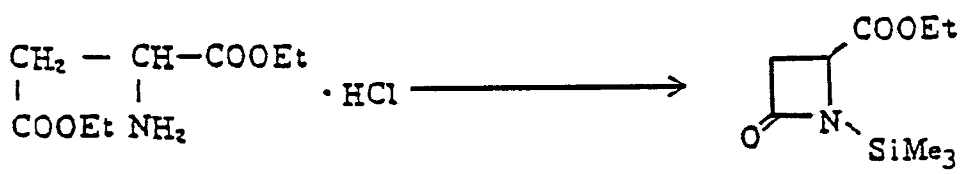

In a mixture of 320 ml of dry THF and 320 ml of dry toluene was suspended 40 g of diethyl L-aspartate hydrochloride under an atmosphere of argon.

The suspension was then ice-cooled. To the suspension 39.5 g of triethyl amine was added dropwise over 10 minutes and then 21.2 g of trimethyl-silyl chloride was added dropwise to the mixture.

After the cooling bath was removed, the mixture was stirred at room temperature for 3 hours. After the mixture was cooled to a temperature of −30 to −20°C, 331 ml of a t-butyl magnesium chloride solution (1.77 mol solution in THF-toluene) was slowly added thereto. The temperature was kept at −20 to −15°C during the addition.

After stirring at a temperature of 0 to 5°C for 2 hours, the reaction mixture was again cooled to a temperature of −30 to −20°C. A solution of 299 ml of 1.96 N hydrochloric acid saturated with ammonium chloride was slowly added to the mixture. During the addition, the internal temperature was kept at −5°C or lower. Insoluble matters were filtered off while washing with ethyl acetate. The filtrate and the washings were combined followed by separation. The aqueous layer was extracted twice with ethyl acetate (200 ml × 2). All of the organic layers were combined and washed 3 times with ice water and then once with saturated aqueous ammonium chloride. After drying over anhydrous magnesium sulfate, the solvent was removed. The residue was distilled under reduced pressure to yield 24.37 g of ethyl (S)-1-trimethylsilyl-4-oxo-2-azetidine-carboxylate. The product showed the following physicochemical properties:

b.p. 85 to 87°C/1 mmHg.

$[\alpha]_D^{22}$ −76.2° (c = 1.4, chloroform).

Elemental analysis for $C_9H_{17}NO_3Si$.

| | C | H | N |
|---|---|---|---|
| Calcd. (%) | 50.20 | 7.96 | 6.50 |
| Found (%) | 49.92 | 7.96 | 6.52 |

NMR ($CDCl_3$) δ ppm: 4.20 (2H, q, $-COOC\underline{H}_2CH_3$),

4.00 (1H, dd, 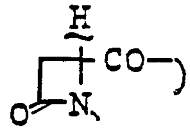), 3.32 (1H, dd, 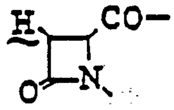),

3.04 (1H, dd, 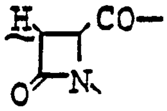),

1.32 (3H, t, —$COOCH_2CH_3$), 0.32 (9H, s, —$SiMe_3$).

IR (neat) $cm^{-1}$: 2950, 1740, 1280, 1190, 840.

Mass spectrum: m/z: 216 (M + 1), 200, 173, 142, 100.

Example 2

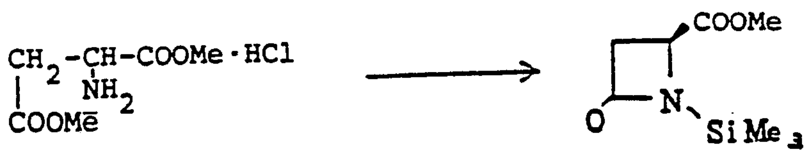

In a manner similar to that in Example 1 except for using 15 g of dimethyl L-aspartate hydrochloride as a raw material, 7.27 g of methyl (S)-1-trimethylsilyl-4-oxo-2-azetidine-carboxylate was obtained.

The product showed the following physicochemical properties.

b.p. 72 to 76°C/1 mmHg.

$[\alpha]_D^{22}$ −73.5° (c = 1.9, chloroform).

NMR ($CDCl_3$) δ ppm:

4.02 (1H, dd, 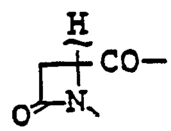),

3.78 (3H, s, $OCH_3$), 3.34 (1H, dd, 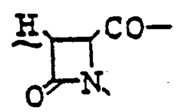),

3.04 (1H, dd, 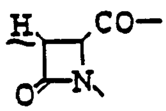),

0.28 (9H, s, $SiMe_3$).

IR (neat) $cm^{-1}$: 2950, 1740, 1280, 1250, 1210, 1180, 840.

Mass spectrum: m/z: 201 ($M^+$), 186, 173, 159, 100.

Example 3

1) 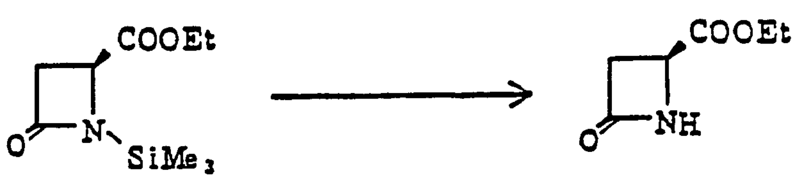

In 40 ml of methanol was dissolved 7.8 g of ethyl (S)-1-trimethylsilyl-4-oxo-2-azetidine-carboxylate obtained in Example 1 followed by addition of 7 ml of Amberlite IR-120 ($H^+$). The mixture was stirred at room temperature for 1.5 hours. The resin was filtered off while washing with methanol. The filtrate was combined with the washings and concentrated under reduced pressure. The residue was distilled under reduced pressure to yield 4.5 g of ethyl (S)-4-oxo-2-azetidine-carboxylate. The product was crystallized by cooling.

The product showed the following physicochemical properties.
b.p. 106 to 110°C/1 mmHg.
m.p. 29 to 32°C.
$[\alpha]_D^{23}$ −42.3° (c = 1.2, chloroform).
Elemental analysis for $C_6H_9NO_3$.

| | C | H | N |
|---|---|---|---|
| Calcd. (%) | 50.35 | 6.34 | 9.79 |
| Found (%) | 50.18 | 6.53 | 9.74 |

NMR ($CDCl_3$) δ ppm: 6.60 (1H, broad s, NH), 4.1~4.4 (3H, q, dd,

$-COOCH_2CH_3$, 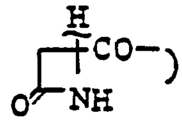),

3.38 (1H, dd, dd, 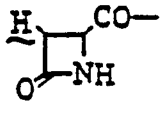), 3.08 (1H, dd, dd,

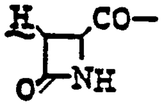),

1.30 (3H, t, $COOCH_2CH_3$).
IR (neat) $cm^{-1}$: 3270, 2960, 1740 (broad), 1370, 1270, 1200, 1050.
Mass spectrum: m/z: 144 (M + 1), 115, 100, 70, 43.

2) 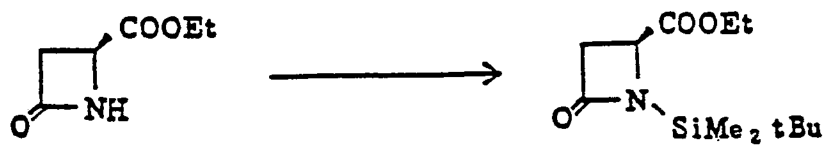

In 40 ml of dry dimethylformamide was dissolved 4.45 g of ethyl (S)-4-oxo-2-azetidine-carboxylate obtained above and the solution was cooled to a temperature of 0 to 5°C. After adding 3.46 g of triethyl amine to the solution, 5.16 g of 5-butyldimethylsilyl chloride was added to the mixture in small portions at a temperature of 0 to 5°C. The mixture was stirred at a temperature of 0 to 5°C for 30 minutes and then at room temperature for 30 minutes. After filtration, the filtrate was concentrated under reduced pressure. The residue was subjected to silica gel (500 ml) column chromatography. By eluting with ethyl acetate- n-hexane (1:1), 7.9 g of ethyl (S)-1-t-butyldimethylsilyl-4-oxo-2-azetidine-carboxylate was obtained. This product was further purified by distillation.

The product showed the following physicochemical properties.
b.p. 93 to 95°C/1 mmHg.
$[\alpha]_D^{24}$ −67.8° (c = 1.3, chloroform).
Elemental analysis for $C_{12}H_{23}NO_3Si$.

| | C | H | N |
|---|---|---|---|
| Calcd. (%) | 55.99 | 9.01 | 5.44 |
| Found (%) | 55.73 | 9.18 | 5.42 |

NMR ($CDCl_3$) δ ppm: 4.22 (2H, q, $COOCH_2CH_3$),

4.02 (1H, dd, 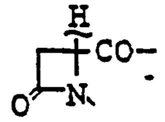), 3.36 (1H, dd, 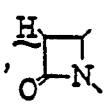),

3.04 (1H, dd, 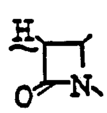),

1.32 (3H, t, $COOCH_2CH_3$), 1.0 (9H, s, tBu), 0.32 (3H, s, Si—$CH_3$), 0.16 (3H, s, Si—$CH_3$).
IR (neat) $cm^{-1}$: 2930, 1750, 1280, 1190, 840, 820.
Mass spectrum: m/z: 258 (M + 1), 216, 200, 172.

**Claims**

1. A process of preparing a 1-substituted silyl-4-oxo-2-azetidine-carboxylic acid lower alkyl ester of general formula (I)

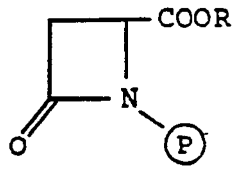

(I)

wherein R represents a $C_1$—$C_5$ alkyl group and Ⓟ represents a protective silyl group substituted with three substituents selected from aryl and $C_1$—$C_5$ alkyl groups, the method comprising silylating the amino group of aspartic acid di-($C_1$—$C_5$) alkyl ester or salt thereof and converting the product to β-lactam form by reaction with Grignard reagent.

2. A process according to claim 1 wherein R represents an ethyl group.

3. A process according to claim 1 for the preparation of ethyl (S) - 1 - trimethylsilyl - 4 - oxo - 2 - azetidine - carboxylate, methyl (S) - 1 - trimethylsilyl - 4 - oxo - 2 - azetidine - carboxylate, ethyl (S) - 1 - t - butyldimethylsilyl - 4 - oxo - 2 - azetidine - carboxylate, ethyl (S) - 1 - dimethylisobutylsilyl - 4 - oxo - 2 - azetidine - carboxylate; ethyl (S) - 1 - diphenylmethylsilyl - 4 - oxo - 2 - azetidine - carboxylate; or ethyl (R) - 1 - trimethylsilyl - 4 - oxo - 2 - azetidine - carboxylate.

**Patentansprüche**

1. Verfahren zur Herstellung eines niederen Alkylesters einer in 1-Stellung substituierten Silyl-4-oxo-2-azetidincarbonsäure der allgemeinen Formel (I)

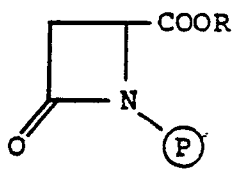

(I)

in der R eine $C_1$—$C_5$-Alkylgruppe und Ⓟ eine Silylschutzgruppe bedeutet, die durch drei Substituenten substituiert ist, ausgewählt aus Aryl- und $C_1$—$C_5$-Alkylgruppen, wobei das Verfahren das Silylieren der Aminogruppe von Aspartinsäure-di-($C_1$—$C_5$)alkylester oder eines Salzes davon und Umwandlung des Produktes in die β-Lactamform durch Umsetzung mit einem Grignard-Reagens umfaßt.

2. Verfahren nach Anspruch 1, wobei R eine Ethylgruppe bedeutet.

3. Verfahren nach Anspruch 1 zur Herstellung von Ethyl - (S) - 1 - trimethylsilyl - 4 - oxo - 2 - azetidin - carboxylat, Methyl - (S) - 1 - trimethylsilyl - 4 - oxo - 2 - azetidin - carboxylat, Ethyl - (S) - 1 - t - butyldimethylsilyl - 4 - oxo - 2 - azetidin - carboxylat, Ethyl - (S) - 1 - dimethylisobutylsilyl - 4 - oxo - 2 - azetidin - carboxylat; Ethyl - (S) - 1 - diphenylmethylsilyl - 4 - oxo - 2 - azetidin - carboxylat oder Ethyl - (R) - 1 - trimethylsilyl - 4 - oxo - 2 - azetidin - carboxylat.

**Revendications**

1. Procédé de fabrication d'un ester d'alkyle inférieur d'acide (silyl substitué)-1 oxo-4 azétidine-2 carboxylique, représenté par la formule générale (I):

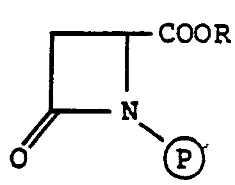

(I)

dans laquelle:

R représente un groupe alkyle en $C_1$—$C_5$; et

Ⓟ représente un groupe silyle protecteur, substitué par trois substituants choisis parmi les groupes aryle et alkyle en $C_1$—$C_5$,

le procédé comprenant la silylation du groupe amino d'un ester de di-(alkyle en $C_1$—$C_5$) d'acide aspartique, ou d'un sel de celui-ci, et la conversion du produit en la forme β-lactame par réaction avec un réactif de Grignard.

2. Procédé selon la revendication 1, dans lequel R représente un groupe éthyle.

3. Procédé selon la revendication 1 pour la fabrication du:
(S)-triméthylsilyl-1 oxo-4 azétidine-2 carboxylate d'éthyle;
(S)-triméthylsilyl-1 oxo-4 azétidine-2 carboxylate d'méthyle;
(S)-t-butyldiméthylsilyl-1 oxo-4 azétidine-2 carboxylate d'ethyle;
(S)-diméthylisobutylsilyl-1 oxo-4 azétidine-2 carboxylate d'ethyle;
(S)-diphénylméthylsilyl-1 oxo-4 azétidine-2 carboxylate d'ethyle; ou
(R)-triméthylsilyl-1 oxo-4 azétidine-2 carboxylate d'ethyle.